# EUROPÄISCHE PATENTSCHRIFT

(11) **EP 2 829 239 B1**
(45) Veröffentlichungstag und Bekanntmachung des Hinweises auf die Patenterteilung: **08.07.2015**
(21) Anmeldenummer: 14177787.0
(22) Anmeldetag: 21.07.2014
(51) Int. Cl.: A61B 17/29, B25J 15/12

(54) **Medizinisches Greifwerkzeug**
Medical gripping tool
Appareil de préhension médical

(30) Priorität: 25.07.2013 DE 102013107972
(43) Veröffentlichungstag der Anmeldung: 28.01.2015
(73) Patentinhaber: Karl Storz GmbH & Co. KG, 78532 Tuttlingen (DE)
(72) Erfinder: Frings, Hermann-Josef, 78532 Tuttlingen (DE)

(56) Entgegenhaltungen:
- EP-A1- 2 502 714
- DE-A1- 10 031 773
- US-A- 4 367 891
- US-A1- 2010 263 500

## Beschreibung

Die vorliegende Erfindung ist auf ein medizinisches Greifwerkzeug, insbesondere für mikroinvasive Eingriffe, bezogen.

Bei medizinischen Eingriffen können Gefäße, Organe, Gewebe oder andere medizinische Objekte oft nicht unmittelbar manuell gegriffen bzw. gehalten werden. Dies gilt insbesondere für mikroinvasive Eingriffe. In diesen Fällen werden Greifwerkzeuge verwendet. Diese stellen zumindest im weiteren Sinne Zangen dar. Für verschiedene Anwendungen, insbesondere Gewebe unterschiedlicher mechanischer Eigenschaften und unterschiedlicher Empfindlichkeit steht ein breites Spektrum unterschiedlicher medizinischer Greifwerkzeuge zur Verfügung, um ein gleichzeitig sicheres und schonendes bzw. atraumatisches Greifen bzw. Halten zu ermöglichen.

In DE 10 2007 026 721 A1 und DE 10 2010 009 259 A1 sind formadaptive medizinische Greifwerkzeuge beschrieben, die auf dem Flossenstrahlen-Effekt (englisch: Fin-Ray-Effect) beruhen. In DE 10 2007 050 018 A1 ist ein medizinisches Greifwerkzeug mit Federelementen in Fluidkammern oder zur Abstützung von Greifflächen beschrieben.

Ein medizinisches Greifwerkzeug, das auf dem Flossenstrahlen-Effekt beruht, kann sich zumindest teilweise an die Gestalt eines gegriffenen bzw. gehaltenen Objekts anpassen. Dadurch kann die Größe der Kontaktfläche zwischen Greifwerkzeug und gegriffenem Objekt vergrößert werden. Ferner wird ein Greifen ermöglicht, das teilweise auf Formschluss beruht. Weitere Verbesserungen sind jedoch wünschenswert, um insbesondere ein noch feinfühligeres Greifen zu ermöglichen. US 2010/0263500 offenbart ein medizinisches Werkzeug gemäß dem Oberbegriff Anspruchs 1.

Eine Aufgabe der vorliegenden Erfindung besteht darin, ein verbessertes medizinisches Greifwerkzeug und ein verbessertes medizinisches Instrument zu schaffen.

Diese Aufgabe wird durch die Gegenstände der unabhängigen Ansprüche gelöst.

Weiterbildungen sind in den abhängigen Ansprüchen angegeben.

Ausführungsformen der vorliegenden Erfindung beruhen auf der Idee, Querverbindungen zwischen den biegeelastischen Holmen einer Branche nicht über Gelenke, sondern über Linearführungen zu schaffen.

Bei einem medizinischen Greifwerkzeug mit mehreren Branchen umfasst jede Branche einen ersten biegeelastischen Holm, einen zweiten biegeelastischen Holm, dessen distales Ende mit dem distalen Ende des ersten biegeelastischen Holms verbunden ist, eine Rippe, die von den proximalen Enden und von den distalen Enden der biegeelastischen Holme beabstandet ist, und eine Linearführung zur Kopplung des zweiten biegeelastischen Holms mit der Rippe derart, dass eine lineare Bewegung des zweiten biegeelastischen Holms relativ zu der Rippe möglich ist.

Das medizinische Greifwerkzeug kann zwei oder mehr Branchen aufweisen, insbesondere drei oder vier Branchen. Alle Branchen des medizinischen Greifwerkzeugs können gleich oder ähnlich ausgebildet sein. Alternativ sind beispielsweise eine oder mehrere Branchen starr, und eine oder mehrere weitere Branchen weisen die beschriebenen Merkmale und insbesondere je mindestens eine Linearführung auf.

Die distalen Enden der beiden biegeelastischen Holme sind insbesondere unmittelbar und starr miteinander verbunden. Insbesondere sind die distalen Enden der biegeelastischen Holme stoffschlüssig (beispielsweise durch Schweißen, Löten oder Kleben) und/oder formschlüssig miteinander verbunden oder werden durch ein gemeinsames Gussteil gebildet. Alternativ können die distalen Enden der biegeelastischen Holme durch ein Gelenk, insbesondere ein Festkörpergelenk, miteinander verbunden sein.

Die Linearführung ermöglicht insbesondere eine Bewegung des zweiten biegeelastischen Holms relativ zu der Rippe in Richtung parallel zu dem zweiten biegeelastischen Holm. Anders ausgedrückt ist der zweite biegeelastische Holm durch die Linearführung in seiner Längsrichtung relativ zu der Rippe bewegbar und senkrecht dazu spiel- und reibungsarm geführt.

Eine Linearführung stellt eine sehr weitgehend miniaturisierbare Alternative zu einem Gelenk dar. Im Gegensatz zu einem ebenfalls sehr weitgehend miniaturisierbaren Festkörpergelenk erzeugt eine Linearführung keine elastischen Kräfte. Die Linearführung kann damit ein noch feinfühligeres Greifen mittels des medizinischen Greifwerkzeugs ermöglichen als ein herkömmliches Festkörpergelenk zwischen Rippe und Holm.

Der zweite biegeelastische Holm und die Rippe können das gleiche oder unterschiedliche Materialien aufweisen. Insbesondere können die Rippe aus Kunststoff (beispielsweise einstückig mit dem ersten biegeelastischen Holm gegossen) und der zweite biegeelastische Holm aus einem Metalldraht gefertigt sein. Da keine miniaturisierten Festkörpergelenke erforderlich sind, können alternativ auch der erste biegeelastische Holm und die Rippe jeweils aus Metall gefertigt sein. Gegenüber einem herkömmlichen auf dem Flossenstrahl-Effekt beruhenden medizinischen Greifwerkzeug, das Festkörpergelenke aufweist und deshalb in der Regel aus Kunststoff gefertigt ist, sind somit bei einem medizinischen Greifwerkzeug mit einer Linearführung andere Materialien verwendbar. Dies kann eine viel weitgehendere Anpassung aller mechanischer Eigenschaften der Branchen des medizinischen Greifwerkzeugs an die aus der vorgesehenen Anwendung resultierenden Anforderungen ermöglichen.

Bei dem medizinischen Greifwerkzeug ist insbesondere der erste biegeelastische Holm an einer Seite der Branche angeordnet, die einem zu greifenden Objekt und der oder den weiteren Branchen zugewandt ist. In diesem Fall ist der zweite biegeelastische Holm an der entgegengesetzten Seite der Branche angeordnet, die von einem zu greifenden Objekt und der oder den weiteren Branchen abgewandt ist. Alternativ ist der zweite biegeelastische Holm an einer Seite der Branche angeordnet, die einem zu greifenden Objekt und der oder den weiteren Branchen zugewandt ist.

Bei einem medizinischen Greifwerkzeug, wie es hier beschrieben ist, sind die Rippe und der erste biegeelastische Holm insbesondere starr miteinander verbunden.

Die Rippe und der erste biegeelastische Holm sind insbesondere ohne ein Festkörpergelenk oder ein anderes Gelenk miteinander verbunden. Die Rippe und der erste biegeelastische Holm können gleichzeitig und einstückig hergestellt sein, beispielsweise mittels eines Gussverfahrens. Alternativ können die Rippe und der erste biegeelastische Holm separat (aus gleichen oder unterschiedlichen Materialien) hergestellt und dann durch Schweißen, Kleben oder auf andere Weise stoff-, form- und/oder kraftschlüssig gefügt sein. Alternativ wird beispielsweise zuerst der erste biegeelastische Holm (aus Metall oder einem anderen Material) gefertigt und dann in eine Gussform eingelegt, in der schließlich die Rippe an den ersten biegeelastischen Holm angeformt wird.

Bei einem medizinischen Greifwerkzeug, wie es hier beschrieben ist, umfasst die Linearführung insbesondere eine Ausnehmung in der Rippe, in der der zweite biegeelastische Holm in seiner Längsrichtung verschiebbar geführt ist.

Die Ausnehmung ist insbesondere ein Kanal bzw. eine Durchgangsbohrung mit einem Querschnitt, der zu dem Querschnitt des zweiten biegeelastischen Holms am Ort der Rippe derart korrespondiert, dass der zweite biegeelastische Holm in dem Kanal bzw. der Durchgangsbohrung spiel- und reibungsarm geführt ist. Alternativ weisen beispielsweise der zweite biegeelastische Holm die Gestalt eines T- oder Doppel-T- bzw. H-Trägers und die Ausnehmung die Gestalt eines (umgekehrten) T auf.

Bei einem medizinischen Greifwerkzeug, wie es hier beschrieben ist, umfasst die Linearführung insbesondere eine Öse, in der der zweite biegeelastische Holm in seiner Längsrichtung verschiebbar geführt ist.

Eine Ausnehmung in der Rippe kann als Öse ausgebildet sein. Alternativ kann die Öse als ringförmige Einrichtung ausgebildet sein. Dabei kann die Öse einstückig mit der Rippe ausgebildet und insbesondere zusammen mit der Rippe monolithisch hergestellt oder als separat hergestelltes und dann an die Rippe gefügtes Bauteil ausgebildet sein.

Bei einem medizinischen Greifwerkzeug, wie es hier beschrieben ist, sind insbesondere mehrere Rippen vorgesehen, die von den proximalen Enden und von den distalen Enden der biegeelastischen Holme und voneinander beabstandet sind, wobei an jeder der mehreren Rippen eine Linearführung, die eine Bewegung des zweiten biegeelastischen Holms relativ zu der Rippe ermöglicht, vorgesehen ist.

Bei einem medizinischen Greifwerkzeug mit mehreren Rippen, wie es hier beschrieben ist, ist insbesondere an einer Rippe der Abstand zwischen der Linearführung und dem ersten biegeelastischen Holm umso kleiner, je kleiner der Abstand der Rippe von den distalen Enden der Holme ist.

Ein medizinisches Greifwerkzeug, wie es hier beschrieben ist, umfasst insbesondere ferner eine Riffelung am ersten biegeelastischen Holm.

Alternativ oder zusätzlich kann ein medizinisches Greifwerkzeug, wie es hier beschrieben ist, eine Riffelung am zweiten biegeelastischen Holm umfassen.

Eine Riffelung an einem der beiden biegeelastischen Holme kann dessen Biegeelastizität erhöhen. Insbesondere eine Riffelung an dem biegeelastischen Holm der einem zu greifenden Objekt und der oder den weiteren Branchen zugewandt ist, kann den Kraftschluss zwischen der Branche und dem zu greifenden Objekt verbessern und/oder durch Formschluss ergänzen.

Bei einem medizinischen Greifwerkzeug, wie es hier beschrieben ist, umfasst der erste biegeelastische Holm insbesondere zumindest entweder zwei biegeelastische Stäbe oder ein streifenförmig-plattenförmiges Bauteil.

Die zwei oder mehr biegeelastischen Stäbe sind insbesondere parallel oder im Wesentlichen parallel (beispielsweise mit nach distal abnehmenden Abständen) angeordnet. Sowohl die biegeelastischen Stäbe als auch das streifenförmig-plattenförmige Bauteil können aus Metall oder einem anderen elastischen Material gebildet sein. Die biegeelastischen Stäbe und/oder das streifenförmig-plattenförmige Bauteil sind insbesondere stoff-, form- und/oder kraftschlüssig mit der Rippe oder den Rippen gefügt. Insbesondere sind die biegeelastische Stäbe bzw. das streifenförmig-plattenförmige Bauteil in einer korrespondierenden Ausnehmung in der Rippe angeordnet und kraftschlüssig (optional: zusätzlich stoff- und/oder formschlüssig) gehalten.

Ein medizinisches Greifwerkzeug, wie es hier beschrieben ist, umfasst insbesondere ferner eine Riffelung am ersten biegeelastischen Holm.

Insbesondere ist der erste biegeelastische Holm in Längsrichtung gewellt. Beispielsweise ist der erste biegeelastische Holm aus einem gewellten Blechstreifen gefertigt. Wenn die biegeelastischen Holme in der Branche so angeordnet sind, dass der zweite biegeelastische Holm an einer Seite der Branche, die einem zu greifenden Objekt und einer oder mehreren Branchen zugewandt ist, angeordnet ist, ist insbesondere anstelle des ersten biegeelastischen Holms der zweite biegeelastische Holm zumindest abschnittsweise mit einer Riffelung oder in Längsrichtung gewellt ausgebildet.

Ein medizinisches Greifwerkzeug, wie es hier beschrieben ist, umfasst insbesondere ferner eine Distanzhülse an dem ersten biegeelastischen Holm, die einen Abstand der Rippe von dem proximalen Ende oder einen Abstand der Rippe von dem distalen Ende des ersten biegeelastischen Holms oder einen Abstand der Rippe von einer weiteren Rippe definiert.

Die Distanzhülse weist insbesondere die Gestalt eines Rohres mit zu dem ersten biegeelastischen Holm korrespondierendem Querschnitt auf. Wenn der erste biegeelastische Holm einen oder mehrere (insbesondere parallele oder im Wesentlichen parallele) Stäbe aufweist, können Distanzhülsen auf einem oder mehreren der Stäbe vorgesehen sein. Die Distanzhülsen sind beispielsweise als Schläuche bzw. flexible Röhrchen aus PTFE oder einem anderen elastischen Material ausgebildet.

Bei der Montage werden im einfachsten Fall abwechselnd Distanzhülsen und Rippen auf den ersten biegeelastischen Holm aufgefädelt. Dadurch kann die Anzahl erforderlicher Schweiß-, Klebe- oder anderer Verbindungen deutlich reduziert werden.

Bei einem medizinischen Greifwerkzeug, wie es hier beschrieben ist, sind insbesondere der erste biegeelastische Holm, der zweite biegeelastische Holm und die Rippe zumindest teilweise mit einem elastischen Material ummantelt.

Insbesondere sind die biegeelastischen Holme und die Rippe oder die Rippen mit Silikon, Gummi, einem Elastomer oder einem anderen elastischen Material teilweise oder vollständig ummantelt. Insbesondere sind die biegeelastischen Holme und die Rippe oder die Rippen in das elastische Material eingegossen.

Die Ummantelung kann mit einer glatten und geschlossenen Oberfläche die Reinigung und Sterilisierung des medizinischen Greifwerkzeugs vereinfachen. Das elastische Material kann ferner zur Schonung von mittels des medizinischen Greifwerkzeugs gegriffenen Objekten beitragen und den Kraftschluss zwischen dem medizinischen Greifwerkzeug und einem gegriffenen Objekt verbessern.

Ein medizinisches Greifwerkzeug, wie es hier beschrieben ist, umfasst insbesondere ferner ein Gelenk zwischen dem proximalen Ende einer Branche und einem Grundkörper des medizinischen Greifwerkzeugs.

Das Gelenk kann eine formschlüssige, spiel- und reibungsarme mechanische Verbindung zwischen der Branche und dem Grundkörper bilden. Alternativ kann das Gelenk als Festkörpergelenk ausgebildet sein. Das Gelenk schafft insbesondere eine gelenkige Verbindung zwischen dem proximalen Ende von einem der beiden biegeelastischen Holme der Branche und dem Grundkörper. Im Falle mehrerer Branchen kann zwischen jeder der Branchen und dem Grundkörper ein Gelenk vorgesehen sein.

Alternativ oder zusätzlich zu einem Gelenk zwischen einer Branche und einem Grundkörper kann ein Gelenk vorgesehen sein, über das die Branche mit einem distalen Ende einer Schub- oder Zugstange oder einer anderen Übertragungseinrichtung derart gekoppelt ist, dass eine Bewegung der Übertragungseinrichtung ein Öffnen oder Schließen des medizinischen Greifwerkzeugs, insbesondere ein Schwenken der Branche bewirkt.

Alternativ kann das Greifwerkzeug derart ausgebildet sein, dass die beiden Branchen zum Öffnen und Schließen parallel oder im Wesentlichen parallel voneinander weg und aufeinander zu bewegt werden können.

Ein medizinisches Greifwerkzeug, wie es hier beschrieben ist, umfasst insbesondere ferner eine Einrichtung zum Bewegen des proximalen Endes des ersten biegeelastischen Holms oder des proximalen Endes des zweiten biegeelastischen Holms.

Die Einrichtung zum Bewegen des proximalen Endes von einem der biegeelastischen Holme kann eine Zug- oder Schubstange oder eine andere Übertragungseinrichtung oder eine Kopplungseinrichtung zur lösbaren oder nicht lösbaren Kupplung mit einer Übertragungseinrichtung sein. Die Einrichtung ist insbesondere zum Verschieben des proximalen Endes von einem der biegeelastischen Holme parallel zu einer Längsachse des medizinischen Greifwerkzeugs oder eines Schafts, mit dem das medizinische Greifwerkzeug verbunden oder verbindbar ist, und/oder parallel zu dem biegeelastischen Holm selbst ausgebildet. Die Einrichtung ist insbesondere als Zugstange ausgebildet und mit dem proximalen Ende des ersten biegeelastischen Holms gekoppelt oder als Schubstange ausgebildet und mit dem proximalen Ende des zweiten biegeelastischen Holms gekoppelt.

Ein medizinisches Instrument umfasst ein medizinisches Greifwerk, wie es hier beschrieben ist.

### Kurzbeschreibung der Figuren

Nachfolgend werden Ausführungsformen anhand der beigefügten Figuren näher erläutert. Es zeigen:
- Figur 1: eine schematische Darstellung eines medizinischen Instruments;
- Figur 2: eine schematische Darstellung eines medizinischen Greifwerkzeugs;
- Figur 3: eine weitere schematische Darstellung des medizinisches Greifwerkzeugs aus Figur 2;
- Figur 4: eine schematische Darstellung eines weiteren medizinischen Greifwerkzeugs;
- Figur 5: eine weitere schematische Darstellung des medizinischen Greifwerkzeugs aus Figur 4;
- Figur 6: eine schematische Darstellung eines Schnitts durch eine Branche eines medizinischen Greifwerkzeugs;
- Figur 7: eine schematische Darstellung eines Schnitts durch eine Branche eines weiteren medizinischen Greifwerkzeugs;
- Figur 8: eine schematische Darstellung einer Branche eines weiteren medizinischen Greifwerkzeugs.

### Beschreibung der Ausführungsformen

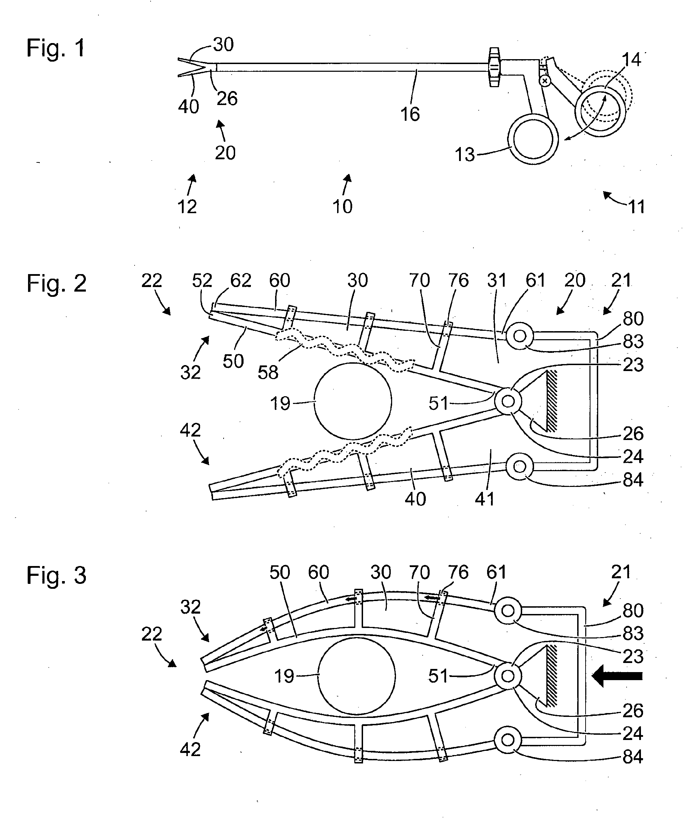

Figur 1 zeigt eine schematische Darstellung eines medizinischen Instruments 10 mit einem proximalen Ende 11 und einem distalen Ende 12. Das medizinische Instrument 10 ist insbesondere für eine Verwendung bei einem mikroinvasiven medizinischen Eingriff ausgebildet. Am proximalen Ende 11 weist das medizinische Instrument ein feststehendes Griffteil 13 und ein bewegbares, insbesondere um eine Schwenkachse, die orthogonal zur Zeichenebene der Figur 1 angeordnet ist, schwenkbares Griffteil 14 auf. Zwischen dem proximalen Ende 11 und dem distalen Ende 12 erstreckt sich ein langer und dünner Schaft 16. Der Schaft 16 kann wie in Figur 1 dargestellt gerade oder gekrümmt, starr oder flexibel sein. Am distalen Ende 12 weist das medizinische Instrument ein medizinisches Greifwerkzeug 20 mit einem Grundkörper 26 und zwei oder mehr Branchen 30, 40 auf.

In dem Schaft 16 ist eine Zug- oder Schubstange oder eine andere Übertragungseinrichtung zum Übertragen einer Bewegung und einer Kraft, die manuell am bewegbaren Griffteil 14 erzeugt wird, zum distalen Ende 12 des medizinischen Instruments 10 angeordnet. Mindestens eine der Branchen 30, 40 ist mit der Übertragungseinrichtung gekoppelt und relativ zum Grundkörper 26 bewegbar, insbesondere um eine Schwenkachse senkrecht zur Längsachse des Schafts 16 und des Grundkörpers 26 schwenkbar. Der Grundkörper 26 ist mit dem distalen Ende des Schafts 16 lösbar über eine Kupplung mechanisch verbunden. Alternativ ist der Grundkörper 26 dauerhaft und nicht zerstörungsfrei lösbar mit dem distalen Ende des Schafts 16 mechanisch verbunden.

Das medizinische Instrument 10 kann weitere und/oder von der Darstellung anhand der Figur 1 abweichende oder in Figur 1 nicht dargestellte Merkmale und Eigenschaften aufweisen. Beispielsweise kann der Schaft 16 zusammen mit dem medizinischen Greifwerkzeug 20 um die Längsachse des Schafts 16 rotierbar sein. Ferner kann das medizinische Instrument 10 zur Vereinfachung einer Reinigung und Sterilisierung in mehrere Teile zerlegbar sein.

Figur 2 zeigt eine schematische Darstellung eines medizinischen Greifwerkzeugs 20, das beispielsweise Bestandteil des anhand der Figur 1 dargestellten medizinischen Instruments 10 oder für dieses ausgebildet sein kann.

Die proximalen Enden 31, 41 der Branchen 30, 40 des medizinischen Greifwerkzeugs 20 sind über Gelenke 23, 24 mit dem in Figur 2 lediglich angedeuteten Grundkörper 26 des medizinischen Greifwerkzeugs 20 gelenkig verbunden. Die Gelenke 23, 24 definieren Schwenkachsen orthogonal zur Zeichenebene der Figur 2. Beide Gelenke 23, 24 können, wie in Figur 2 angedeutet, miteinander kombiniert oder ineinander verschachtelt sein, so dass die Schwenkachsen beider Branchen 30, 40 identisch sind. Alternativ und abweichend von der Darstellung in Figur 2 können die beiden Gelenke 23, 24 nebeneinander oder voneinander beabstandet angeordnet sein und zwei beabstandete und insbesondere parallele Schwenkachsen definieren.

Am proximalen Ende 21 des medizinischen Greifwerkzeugs 20 ist ferner eine Kopplungseinrichtung 80 angedeutet, die mittels zweier Gelenke 83, 84 mit den Branchen 30, 40 gelenkig verbunden ist. Die Kopplungseinrichtung 80 ist parallel zur Längsachse eines Schafts 16 (vgl. Figur 1), der mit dem medizinischen Greifwerkzeug 20 verbunden oder verbindbar ist, verschiebbar. Die Kopplungseinrichtung 80 ist mittels einer nicht dargestellten Kupplung oder unmittelbar und untrennbar mit einer Schubstange in dem Schaft 16 (vgl. Figur 1) verbunden. Durch den Abstand der Gelenke 83, 84 zwischen der Kopplungseinrichtung und den Branchen 30, 40 einerseits und den Gelenken 23, 24 zwischen dem Grundkörper 26 und den Branchen 30, 40 anderseits bewirkt eine Bewegung der Kopplungseinrichtung 80 parallel zur Längsachse des Schafts 16 (vgl. Figur 1) ein Schwenken der Branchen 30, 40 um die durch die Gelenke 23, 24 definierten Schwenkachsen.

Die Branchen 30, 40 sind einander entsprechend und symmetrisch zueinander oder im Wesentlichen symmetrisch zueinander ausgebildet. Deshalb sind nachfolgend nur Merkmale und Eigenschaften der ersten Branche 30 beschrieben.

Das proximale Ende 31 der ersten Branche 30 ist wie bereits beschrieben über das Gelenk 23 mit dem Grundkörper 26 und über das Gelenk 83 mit der Kopplungseinrichtung 80 verbunden. Das distale Ende 32 der ersten Branche 30 und das distale Ende 42 der zweiten Branche 40 bilden das distale Ende 22 des medizinischen Greifwerkzeugs 20.

Die erste Branche 30 umfasst einen ersten biegeelastischen Holm 50 und einen zweiten biegeelastischen Holm 60. Das proximale Ende 51 des ersten biegeelastischen Holms 50 ist über das Gelenk 23 mit dem Grundkörper 26 des medizinischen Greifwerkzeugs 20 verbunden. Das proximale Ende 61 des zweiten biegeelastischen Holms 60 ist über das Gelenk 83 mit der Kopplungseinrichtung 80 verbunden.

Die distalen Enden 52, 62 der biegeelastischen Holme 50, 60 bilden das distale Ende 32 der ersten Branche 30 des medizinischen Greifwerkzeugs 20. Die distalen Enden 52, 62 der biegeelastischen Holme 50, 60 sind miteinander starr oder gelenkig verbunden, insbesondere durch eine Schweißung, eine Klebung oder mittels eines in Figur 2 nicht dargestellten Verbindungsbauteils.

Mehrere Rippen 70 sind jeweils mit dem ersten biegeelastischen Holm 50 starr oder im Wesentlichen starr verbunden. Jede Rippe 70 weist nahe einem vom ersten biegeelastischen Holm 50 beabstandeten Ende oder Rand eine Ausnehmungs- bzw. Durchgangsbohrung 76 auf. Der zweite biegeelastische Holm 60 erstreckt sich durch die Ausnehmungen 76 aller Rippen 70 hindurch. Die Querschnitte der Lumen der Ausnehmungen 76 und die Querschnitte des zweiten biegeelastischen Holms 60 in den Bereichen, in denen der zweite biegeelastische Holm 60 in der jeweiligen Ausnehmung 76 angeordnet ist, entsprechen einander so weitgehend, dass der zweite biegeelastische Holm 60 in den Ausnehmungen 76 der Rippen 70 spiel- und reibungsarm geführt ist.

Wie nachfolgend anhand der Figur 3 beschrieben ist, ist der zweite biegeelastische Holm 60 in seiner Längsrichtung in den Ausnehmungen 76 bewegbar, wobei eine Bewegung des zweiten biegeelastischen Holms 60 in den Ausnehmungen 76 mit einer elastischen Verformung der biegeelastischen Holme 50, 60 und damit der gesamten ersten Branche 30 einhergeht.

In Figur 2 ist das medizinische Greifwerkzeug 20 in einer offenen Konfiguration gezeigt. Zwischen den Branchen 30, 40 ist ein mittels des medizinischen Greifwerkzeugs 20 greifbares Objekt 19 angedeutet. Die Branchen 30, 40 sind unverformt, die biegeelastischen Holme 50, 60 sind gerade. Um das Greifen des Objekts 19 zu vereinfachen und das Risiko eines Abrutschens zu mindern, kann der erste biegeelastische Holm 50 abweichend von dem in Figur 2 in durchgezogenen Linien Angedeuteten eine Riffelung 58, die in Figur 2 in gestrichelten Linien angedeutet ist, aufweisen. Die Riffelung 58 entsteht insbesondere durch eine Wellenform bzw. eine Wellung des ersten biegeelastischen Holms 50 in Längsrichtung.

Figur 3 zeigt eine weitere schematische Darstellung des medizinischen Greifwerkzeugs 20 aus Figur 2. Abweichend von der Darstellung in Figur 2 ist das medizinische Greifwerkzeug 20 in Figur 3 in einer geschlossenen bzw. das Objekt 19 greifenden Konfiguration dargestellt. Durch einen großen horizontalen Pfeil ist eine Kraft angedeutet, durch die die Kopplungseinrichtung 80 und mit ihr die Gelenke 83, 84 relativ zum Grundkörper 26 und zu den Gelenken 23, 24 nach distal (in den Figuren: links) verschoben sind. Die Branchen 30, 40 sind dadurch so weit an das Objekt 19 heran geschwenkt, dass die ersten biegeelastischen Holme 50 das Objekt 19 berühren. Aufgrund der nach distal schiebenden Kraft an der Kopplungseinrichtung 80 einerseits und der zwischen dem Objekt 19 und den ersten biegeelastischen Holmen 50 wirkenden Kraft andererseits sind die biegeelastischen Holme 50, 60 elastisch verformt und der zweite biegeelastische Holm 60 in den Ausnehmungen 76 in den Rippen 70 relativ zu diesen nach distal verschoben. Die Verschiebung des zweiten biegeelastischen Holms 60 der ersten Branche 30 relativ zu den Ausnehmungen 76 der Rippen 70 ist in Figur 3 durch drei kleine Pfeile angedeutet. Im Ergebnis umschließen die Branchen 30, 40 des medizinischen Greifwerkzeugs 20 das Objekt 19 zumindest teilweise. Das Objekt 19 wird durch die Branchen 30, 40 nicht nur reib- bzw. kraftschlüssig, sondern auch formschlüssig gehalten.

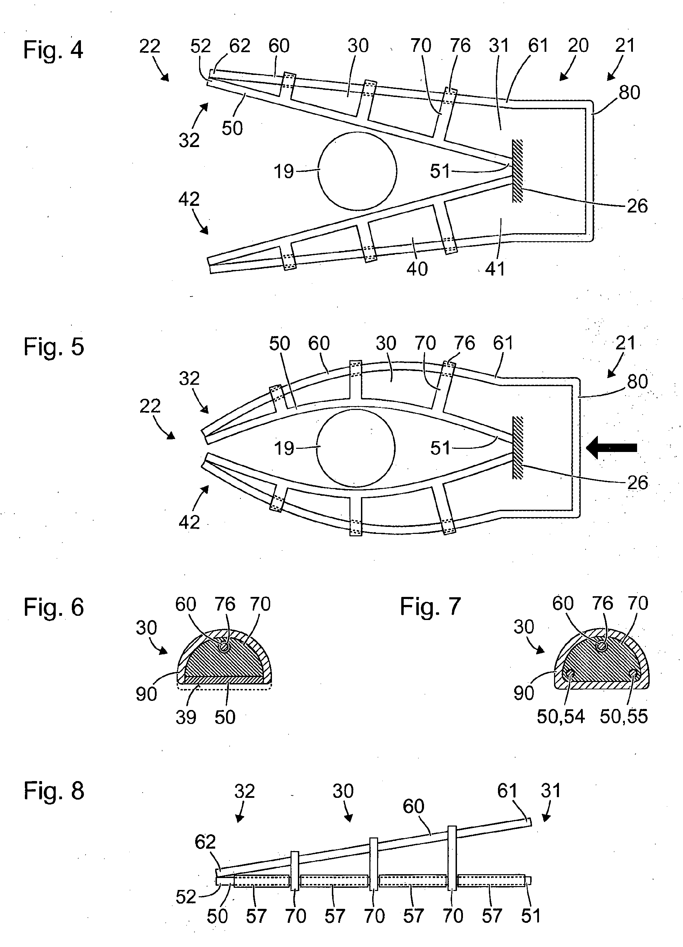

Figur 4 zeigt eine schematische Darstellung eines weiteren medizinischen Greifwerkzeugs 20, das in einigen Merkmalen und Eigenschaften dem oben anhand der Figuren 2 und 3 dargestellten medizinischen Greifwerkzeug ähnelt. Nachfolgend sind Merkmale und Eigenschaften beschrieben, in denen das in Figur 4 dargestellte medizinische Greifwerkzeug 20 sich von dem oben anhand der Figuren 2 und 3 dargestellten medizinischen Greifwerkzeug unterscheidet.

Das in Figur 4 dargestellte medizinische Greifwerkzeug unterscheidet sich von dem anhand der Figuren 2 und 3 dargestellten medizinischen Greifwerkzeug insbesondere dadurch, dass zwischen dem Grundkörper 26 einerseits und den Branchen 30, 40 bzw. den ersten biegeelastischen Holmen 50 andererseits keine Gelenke vorgesehen sind. Ein Öffnen und Schließen der Branchen 30, 40 erfolgt stattdessen durch elastische Verformung der biegeelastischen Holme 50 der Branchen 30, 40 nahe dem Grundkörper 26.

Das in Figur 4 dargestellte medizinische Greifwerkzeug 20 unterscheidet sich von dem oben anhand der Figuren 2 und 3 dargestellten medizinischen Greifwerkzeug ferner dadurch, dass zwischen der Kopplungseinrichtung 80 einerseits und den Branchen 30, 40 bzw. den zweiten biegeelastischen Holmen 60 der Branchen 30, 40 andererseits keine Gelenke vorgesehen sind. Stattdessen werden beim Öffnen und Schließen der Branchen 30, 40 die zweiten biegeelastischen Holme 60 der Branchen 30, 40 nahe ihren proximalen Enden 61 elastisch verformt. Alternativ oder zusätzlich kann die Kopplungseinrichtung 80 elastisch verformt werden.

Figur 5 zeigt eine weitere schematische Darstellung des medizinischen Greifwerkzeugs aus Figur 4. Ähnlich wie Figur 3 zeigt Figur 5 das medizinische Greifwerkzeug 20 in einer geschlossenen bzw. greifenden Konfiguration. Durch eine durch einen großen horizontalen Pfeil angedeutete Kraft ist die Kopplungseinrichtung 80 nach distal verschoben. Die biegeelastischen Holme 50, 60 der Branchen 30, 40 sind elastisch verformt, die zweiten biegeelastischen Holme 60 der Branchen 30, 40 sind relativ zu den Ausnehmungen 76 in den Rippen 70 nach distal verschoben. Das medizinische Greifwerkzeug 20 greift und hält das Objekt 19 teilweise formschlüssig.

Abweichend von den Darstellungen in den Figuren 2 bis 5 können beispielsweise die proximalen Enden 51 der ersten biegeelastischen Holme 50 über Gelenke 23, 24 mit einem Grundkörper 26 und die proximalen Enden 61 der zweiten biegeelastischen Holme 60 ohne Gelenk oder über ein Festkörpergelenk mit der Kopplungseinrichtung 80 verbunden sein.

Abweichend von den Darstellungen anhand der Figuren 2 bis 5 können die proximalen Enden 51 der ersten biegeelastischen Holme 50 ohne ein Gelenk mit einem Grundkörper 26 und die proximalen Enden 61 der zweiten biegeelastischen Holme 60 über Gelenke mit einer Kopplungseinrichtung 80 verbunden sein.

Abweichend von der Darstellung anhand der Figuren 2 bis 5 können die proximalen Enden 61 der zweiten biegeelastischen Holme 60 (unmittelbar oder über Gelenke) mit dem Grundkörper 26 und die proximalen Enden 51 der ersten biegeelastischen Holme 50 (unmittelbar oder über Gelenke) mit einer Übertragungseinrichtung 80 verbunden sein. In diesem Fall muss eine Übertragungseinrichtung zum Schließen der Branchen 30, 40 eine Zugkraft übertragen.

Abweichend von der Darstellung anhand der Figuren 2 bis 5 kann eine Branche starr und die andere Branche mittels der Übertragungseinrichtung 80 bewegbar sein. Abweichend von der Darstellung anhand der Figuren 2 bis 5 können mehr als zwei Branchen vorgesehen sein, von denen insbesondere höchstens eine starr ausgebildet ist, und von denen insbesondere mindestens eine schwenkbar ausgebildet ist.

Figur 6 zeigt eine schematische Darstellung eines Schnitts durch eine Branche 30 eines medizinischen Greifwerkzeugs 20, wie es oben anhand der Figuren 2 bis 5 dargestellt wurde. Die Schnittebene der Figur 6 ist orthogonal zu den Zeichenebenen der Figuren 1 bis 5 und orthogonal oder im Wesentlichen orthogonal zum ersten biegeelastischen Holm 50 und zum zweiten biegeelastischen Holm 60. Die Schnittebene der Figur 6 schneidet eine Rippe 70 der Branche 30.

In Figur 6 ist erkennbar, dass der erste biegeelastische Holm 50 einen flachen rechteckigen Querschnitt aufweist. Die Breite des ersten biegeelastischen Holms 50 entspricht fast der Gesamtbreite der Branche 30 in der in Figur 6 dargestellten Schnittebene. Die in Richtung orthogonal zur Breite und damit in Figur 6 in senkrechter Richtung gemessene Höhe des Querschnitts des ersten biegeelastischen Holms 5 ist wesentlich kleiner als seine Breite.

Die Rippe 70 weist insbesondere die Gestalt einer halbkreisförmigen Platte auf, die sich im Wesentlichen senkrecht zum ersten biegeelastischen Holm 50 und parallel zur Zeichenebene der Figur 6 erstreckt. Die Rippe 70 ist mit dem ersten biegeelastischen Holm 50-insbesondere durch Schweißen, Löten oder Kleben - stoffschlüssig gefügt. Alternativ oder zusätzlich kann die Rippe 70 mit dem ersten biegeelastischen Holm 50 form- und/oder kraftschlüssig gefügt oder mit ihm einstückig und gleichzeitig gefertigt (beispielsweise gegossen) sein.

Der zweite biegeelastische Holm 60 weist die Gestalt eines dünnen Stabs und insbesondere einen kreisförmigen Querschnitt auf. Der Querschnitt des Lumens der Ausnehmung 76 ähnelt dem Querschnitt des zweiten biegeelastischen Holms 60, insbesondere sind beide kreisförmig. Der Querschnitt des Lumens der Ausnehmung 76 ist etwas größer als der Querschnitt des zweiten biegeelastischen Holms 60, so dass der zweite biegeelastische Holm 60 in der Ausnehmung 76 spiel- und reibungsarm geführt ist. Dadurch kann der zweite biegeelastische Holm 60 in seiner Längsrichtung (orthogonal oder im Wesentlichen orthogonal zur Schnittebene der Figur 6) relativ zur Rippe 70 bewegt werden.

Die Branche 30 weist eine Ummantelung 90 auf, die abgesehen von einer Greiffläche 39 am ersten biegeelastischen Holm 50 die Branche 30 vollständig umschließt. Alternativ kann die Ummantelung 90, wie in Figur 6 durch eine gestrichelte Linie angedeutet, die Branche 30 vollständig umschließen und auch die Greiffläche der Branche 30 bilden. Die Ummantelung 90 weist ein elastisches Material auf, beispielsweise Silikon, Gummi oder ein Elastomer. Die Ummantelung kann - insbesondere wenn sie auch die Greiffläche 39 bildet - die atraumatischen Eigenschaften des medizinischen Greifwerkzeugs verbessern und die Haftreibung zwischen der Branche 30 und einem zu greifenden Objekt 19 (vgl. Figuren 2 bis 4) verbessern. Ferner kann die Ummantelung 90 mit einer geschlossenen und glatten Oberfläche die Reinigung und Sterilisierung des medizinischen Greifwerkzeugs vereinfachen.

Figur 7 zeigt eine schematische Darstellung eines Schnitts durch eine Branche 30 eines weiteren Greifwerkzeugs, das in einigen Merkmalen und Eigenschaften den oben anhand der Figuren 1 bis 6 dargestellten medizinischen Greifwerkzeugen ähnelt. Die Schnittebene der Figur 7 ist orthogonal zu den Schnittebenen der Figuren 1 bis 5, orthogonal oder im Wesentlichen orthogonal zu den biegeelastischen Holmen 50, 60 und entspricht der Schnittebene der Figur 6.

Bei der in Figur 7 dargestellten Branche 30 wird der erste biegeelastische Holm 50 durch zwei parallele oder im Wesentlichen parallele biegeelastische Stäbe 54, 55 gebildet. Die biegeelastischen Stäbe 54, 55 sind ähnlich wie der zweite biegeelastische Holm 60 in Ausnehmungen korrespondierender Querschnitte in der Rippe 70 angeordnet. Die Querschnitte der Ausnehmungen in der Rippe 70, in denen die biegeelastischen Stäbe 54, 55 angeordnet sind, entsprechen jedoch den Querschnitten der biegeelastischen Stäbe 54, 55 derart, dass die biegeelastischen Stäbe 54, 55 reib- bzw. kraftschlüssig mit der Rippe 70 verbunden sind. Alternativ oder zusätzlich können die Stäbe 54, 55 mit der Rippe 70 stoff- und/oder formschlüssig gefügt sein.

Bei der in Figur 7 gezeigten Branche 30 umschließt die Ummantelung 90, ähnlich wie bereits anhand der Figur 6 dargestellt, die Branche 30 vollständig.

Figur 8 zeigt eine schematische Darstellung einer Branche 30 eines weiteren medizinischen Greifwerkzeugs, das in einigen Merkmalen und Eigenschaften den oben anhand der Figuren 1 bis 5 dargestellten medizinischen Greifwerkzeugen ähnelt. Die Zeichenebene der Figur 8 entspricht den Zeichenebenen der Figuren 1 bis 5. Im Unterschied zu den Darstellungen in den Figuren 2 bis 5 sind in Figur 8 weder eine zweite Branche noch eine Verbindung der ersten Branche 30 mit einem Grundkörper oder einer Kopplungseinrichtung dargestellt. Sowohl das proximale Ende 51 des ersten biegeelastischen Holms 50 als auch das proximale Ende 61 des zweiten biegeelastischen Holms 60 kann jeweils über ein Gelenk (oder mehrere Gelenke) oder unmittelbar ohne ein Gelenk mit einem Grundkörper 26 bzw. einer Kopplungseinrichtung 80 (vgl. Figuren 2 bis 5) verbunden sein.

Abweichend von den oben anhand der Figuren 1 bis 6 dargestellten medizinischen Greifwerkzeugen und ähnlich wie anhand der Figur 7 dargestellt, ist der erste biegeelastische Holm 50 in Ausnehmungen in den Rippen 70 angeordnet. Der Holm 50 umfasst beispielsweise, ähnlich wie anhand der Figur 7 dargestellt, zwei oder mehr parallele oder im Wesentlichen parallele biegeelastische Stäbe.

Abweichend von der Darstellung anhand der Figur 7 sind bei dem in Figur 8 gezeigten Beispiel die Rippen 70 mit dem ersten biegeelastischen Holm 50 nicht unmittelbar starr verbunden, sondern an sich parallel zum ersten biegeelastischen Holm 50 verschiebbar. Um eine Verschiebung der Rippen 70 relativ zum ersten biegeelastischen Holm 50 zu verhindern, sind Distanzhülsen 57 zwischen den Rippen 70 und zwischen den Rippen 70 und den proximalen und distalen Enden 51, 52 des ersten biegeelastischen Holms 50 vorgesehen. Die Distanzhülsen 57 umschließen den biegeelastischen Holm 50 oder dessen Bestandteile (beispielsweise biegeelastische Stäbe) insbesondere rohrförmig. Um die Biegeelastizität des ersten biegeelastischen Holms 50 zu erhalten, sind die Distanzhülsen 57 ebenfalls biegeelastisch.

Zur Fertigung können die Distanzhülsen 57 und die Rippen 70 abwechselnd auf den Holm 50 aufgefädelt werden. Durch das ohnehin erforderliche Fügen der distalen Enden 52, 62 der biegeelastischen Holme 50, 60 miteinander und der proximalen Enden 51, 61 der biegeelastischen Holme 50, 60 mit Gelenken, einem Grundkörper 26 bzw. einer Kopplungseinrichtung 80 (vgl. Figuren 2 bis 5) werden die Distanzhülsen 57 und die Rippen 70 beidseitig gehalten und können nicht mehr entlang des ersten biegeelastischen Holms 50 bewegt werden.

### Bezugszeichen

10 medizinisches Instrument
11 proximales Ende des medizinischen Instruments 10
12 distales Ende des medizinischen Instruments 10
13 feststehendes Griffteil am proximalen Ende des medizinischen Instruments 10
14 bewegbares (insb. schwenkbares) Griffteil am proximalen Ende 11 des medizinischen Instruments 10
16 Schaft des medizinischen Instruments 10
19 mittels des medizinischen Instruments 10 greifbares Objekt
20 medizinisches Greifwerkzeug
21 proximales Ende des medizinischen Greifwerkzeugs 20
22 distales Ende des medizinischen Greifwerkzeugs 20
23 Gelenk zwischen der ersten Branche 30 und dem Grundkörper 26 des medizinischen Greifwerkzeugs 20
24 Gelenk zwischen der zweiten Branche 40 und dem Grundkörper 26 des medizinischen Greifwerkzeugs 20
26 Grundkörper
30 erste Branche des medizinischen Greifwerkzeugs 20
31 proximales Ende der ersten Branche 30
32 distales Ende der ersten Branche 30
39 Greiffläche der ersten Branche 30
40 zweite Branche des medizinischen Greifwerkzeugs 20
41 proximales Ende der zweiten Branche 40
42 distales Ende der zweiten Branche 40
50 erster biegeelastischer Holm der ersten Branche 30
51 proximales Ende des ersten biegeelastischen Holms 50
52 distales Ende des ersten biegeelastischen Holms 50
54 erster biegeelastischer Stab des ersten Holms 50
55 zweiter biegeelastischer Stab des ersten Holms 50
57 Distanzhülse am ersten biegeelastischen Holm 50
58 Riffelung am ersten biegeelastischen Holm 50
60 zweiter biegeelastischer Holm der ersten Branche 30
61 proximales Ende des zweiten biegeelastischen Holms 60
62 distales Ende des zweiten biegeelastischen Holms 60
70 Rippe zwischen dem ersten biegeelastischen Holm 50 und dem zweiten biegeelastischen Holms 60
76 Ausnehmung in der Rippe 50 für den zweiten biegeelastischen Holm 60
80 Kopplungseinrichtung
83 Gelenk zwischen Kopplungseinrichtung 80 und erster Branche 30
84 Gelenk zwischen Kopplungseinrichtung 80 und zweiter Branche 40
90 Ummantelung der biegeelastischen Holme 50, 60 und der Rippe 70

## Patentansprüche

1. Medizinisches Greifwerkzeug (20), mit mehreren Branchen (30, 40), wobei eine Branche (30) umfasst:
einen ersten biegeelastischen Holm (50)
einen zweiten biegeelastischen Holm (60), dessen distales Ende (62) mit dem distalen Ende (52) des ersten biegeelastischen Holms (50) verbunden ist;
eine Rippe (70), die von den proximalen Enden (51, 61) und von den distalen Enden (52, 62) der biegeelastischen Holme (50, 60) beabstandet ist;
**dadurch gekennzeichnet daß**,
eine Linearführung (76) zur Kopplung des zweiten biegeelastischen Holms (60) mit der Rippe (70) derart vorgesehen ist, dass eine lineare Bewegung des zweiten biegeelastischen Holms (60) relativ zu der Rippe (70) möglich ist.

2. Medizinisches Greifwerkzeug (20) nach dem vorangehenden Anspruch, bei dem die Rippe (70) und der erste biegeelastische Holm (50) starr miteinander verbunden sind.

3. Medizinisches Greifwerkzeug (20) nach einem der vorangehenden Ansprüche, bei dem die Linearführung eine Ausnehmung (76) in der Rippe (70) umfasst, in der der zweite biegeelastische Holm (60) in seiner Längsrichtung verschiebbar geführt ist.

4. Medizinisches Greifwerkzeug (20) nach einem der vorangehenden Ansprüche, bei dem die Linearführung eine Öse umfasst, in der der zweite biegeelastische Holm (60) in seiner Längsrichtung verschiebbar geführt ist.

5. Medizinisches Greifwerkzeug (20) nach einem der vorangehenden Ansprüche, bei dem mehrere Rippen (70) vorgesehen sind, die von den proximalen Enden (51, 61) und von den distalen Enden (52, 62) der biegeelastischen Holme (50, 60) und voneinander beabstandet sind, wobei an jeder der mehreren Rippen (70) eine Linearführung (76), die eine Bewegung des zweiten biegeelastischen Holms (60) relativ zu der Rippe (70) ermöglicht, vorgesehen ist.

6. Medizinisches Greifwerkzeug (20) nach einem der vorangehenden Ansprüche, ferner mit:
einer Riffelung (58) am ersten biegeelastischen Holm (50).

7. Medizinisches Greifwerkzeug (20) nach einem der vorangehenden Ansprüche, bei dem der erste biegeelastische Holm (50) zumindest entweder zwei biegeelastische Stäbe (54, 55) oder einen streifenförmig-plattenförmige Bauteil umfasst.

8. Medizinisches Greifwerkzeug (20) nach einem der vorangehenden Ansprüche, ferner mit:
einer Distanzhülse (57) an dem ersten biegeelastischen Holm (50), die einen Abstand der Rippe (70) von dem proximalen Ende (51) oder einen Abstand der Rippe (70) von dem distalen Ende (52) des ersten biegeelastischen Holm (50) oder einen Abstand der Rippe (70) von einer weiteren Rippe (70) definiert.

9. Medizinisches Greifwerkzeug (20) nach einem der vorangehenden Ansprüche, bei dem der erste biegeelastische Holm (50), der zweite biegeelastische Holm (60) und die Rippe (70) zumindest teilweise mit einem elastischen Material ummantelt sind.

10. Medizinisches Greifwerkzeug (20) nach einem der vorangehenden Ansprüche, ferner mit:
einem Gelenk (23, 24) zwischen dem proximalen Ende (31, 41) einer Branche (30, 40) und einem Grundkörper (26).

11. Medizinisches Greifwerkzeug (20) nach einem der vorangehenden Ansprüche, ferner mit:
einer Einrichtung (80) zum Bewegen des proximalen Endes des ersten biegeelastischen Holms (50) oder des proximalen Endes des zweiten biegeelastischen Holms (60).

12. Medizinisches Instrument (10) mit:
einem medizinischen Greifwerkzeug (20) gemäße einem der vorangehenden Ansprüche.

## Claims

1. Medical gripping tool (20) with multiple branches (30, 40), wherein one branch (30) comprises:
a first flexurally elastic spar (50);
a second flexurally elastic spar (60), of which the distal end (62) is connected to the distal end (52) of the first flexurally elastic spar (50);
a rib (70), which is spaced apart from the proximal ends (51, 61) and from the distal ends (52, 62) of the flexurally elastic spars (50, 60);
**characterized in that** a linear guide (76) is provided for coupling the second flexurally elastic spar (60) to the rib (70), in such a way that a linear movement of the second flexurally elastic spar (60) relative to the rib (70) is possible.

2. Medical gripping tool (20) according to the preceding claim, in which the rib (70) and the first flexurally elastic spar (50) are connected rigidly to each other.

3. Medical gripping tool (20) according to one of the preceding claims, in which the linear guide comprises a recess (76) in the rib (70), in which recess (76) the second flexurally elastic spar (60) is guided displaceably in its longitudinal direction.

4. Medical gripping tool (20) according to one of the preceding claims, in which the linear guide comprises an eyelet in which the second flexurally elastic spar (60) is guided displaceably in its longitudinal direction.

5. Medical gripping tool (20) according to one of the preceding claims, in which several ribs (70) are provided which are spaced apart from the proximal ends (51, 61) and from the distal ends (52, 62) of the flexurally elastic spars (50, 60) and from each other, wherein on each of the several ribs (70) a linear guide (76) is provided which permits a movement of the second flexurally elastic spar (60) relative to the rib (70).

6. Medical gripping tool (20) according to one of the preceding claims, also with:
a fluting (58) on the first flexurally elastic spar (50).

7. Medical gripping tool (20) according to one of the preceding claims, in which the first flexurally elastic spar (50) comprises at least either two flexurally elastic rods (54, 55) or one strip-shaped/plate-shaped component.

8. Medical gripping tool (20) according to one of the preceding claims, also with:
a spacer sleeve (57) on the first flexurally elastic spar (50), which spacer sleeve (57) defines a distance of the rib (70) from the proximal end (51) or a distance of the rib (70) from the distal end (52) of the first flexurally elastic spar (50) or a distance of the rib (70) from a further rib (70).

9. Medical gripping tool (20) according to one of the preceding claims, in which the first flexurally elastic spar (50), the second flexurally elastic spar (60) and the rib (70) are at least partially encapsulated by an elastic material.

10. Medical gripping tool (20) according to one of the preceding claims, also with:
a hinge (23, 24) between the proximal end (31, 41) of a branch (30, 40) and a main body (26).

11. Medical gripping tool (20) according to one of the preceding claims, also with:
a mechanism (80) for moving the proximal end of the first flexurally elastic spar (50) or the proximal end of the second flexurally elastic spar (60).

12. Medical instrument (10) with:
a medical gripping tool (20) according to one of the preceding claims.

## Revendications

1. Appareil de préhension médical (20) comprenant plusieurs branches (30, 40), une branche (30) comprenant :
un premier longeron élastiquement flexible (50), un deuxième longeron élastiquement flexible (60) dont l'extrémité distale (62) est connectée à l'extrémité distale (52) du premier longeron élastiquement flexible (50) ;
une nervure (70) qui est espacée des extrémités proximales (51, 61) et des extrémités distales (52, 62) des longerons élastiquement flexibles (50, 60) ;
**caractérisé en ce**
**qu'**un guide linéaire (76) pour l'accouplement du deuxième longeron élastiquement flexible (60) à la nervure (70) est prévu de telle sorte qu'un déplacement linéaire du deuxième longeron élastiquement flexible (60) par rapport à la nervure (70) soit possible.

2. Appareil de préhension médical (20) selon la revendication précédente, dans lequel la nervure (70) et le premier longeron élastiquement flexible (50) sont connectés rigidement l'un à l'autre.

3. Appareil de préhension médical (20) selon l'une quelconque des revendications précédentes, dans lequel le guide linéaire comprend un évidement (76) dans la nervure (70), dans lequel est guidé le deuxième longeron élastiquement flexible (60) de manière déplaçable dans sa direction longitudinale.

4. Appareil de préhension médical (20) selon l'une quelconque des revendications précédentes, dans lequel le guide linéaire comprend un oeillet dans lequel est guidé le deuxième longeron élastiquement flexible (60) de manière déplaçable dans sa direction longitudinale.

5. Appareil de préhension médical (20) selon l'une quelconque des revendications précédentes, dans lequel plusieurs nervures (70) sont prévues, lesquelles sont espacées des extrémités proximales (51, 61) et des extrémités distales (52, 62) des longerons élastiquement flexibles (50, 60) et les unes des autres, un guide linéaire (76) qui permet un déplacement du deuxième longeron élastiquement flexible (60) par rapport à la nervure (70) étant prévu au niveau de chacune des plusieurs nervures (70).

6. Appareil de préhension médical (20) selon l'une quelconque des revendications précédentes, présentant en outre :
un nervurage (58) au niveau du premier longeron élastiquement flexible (50).

7. Appareil de préhension médical (20) selon l'une quelconque des revendications précédentes, dans lequel le premier longeron élastiquement flexible (50) comprend au moins soit deux barres élastiquement flexibles (54, 55) soit un composant en forme de plaque en forme de bande.

8. Appareil de préhension médical (20) selon l'une quelconque des revendications précédentes, comprenant en outre :
une douille d'espacement (57) au niveau du premier longeron élastiquement flexible (50), laquelle définit un espacement de la nervure (70) de l'extrémité proximale (51) ou un espacement de la nervure (70) de l'extrémité distale (52) du premier longeron élastiquement flexible (50) ou un espacement de la nervure (70) d'une autre nervure (70).

9. Appareil de préhension médical (20) selon l'une quelconque des revendications précédentes, dans lequel le premier longeron élastiquement flexible (50), le deuxième longeron élastiquement flexible (60) et la nervure (70) sont au moins en partie enrobés par un matériau élastique.

10. Appareil de préhension médical (20) selon l'une quelconque des revendications précédentes, comprenant en outre :
une articulation (23, 24) entre l'extrémité proximale (31, 41) d'une branche (30, 40) et un corps de base (26).

11. Appareil de préhension médical (20) selon l'une quelconque des revendications précédentes, comprenant en outre :
un dispositif (80) pour déplacer l'extrémité proximale du premier longeron élastiquement flexible (50) ou l'extrémité proximale du deuxième longeron élastiquement flexible (60).

12. Instrument médical (10), comprenant :
un appareil de préhension médical (20) selon l'une quelconque des revendications précédentes.
